# EUROPEAN PATENT SPECIFICATION

(11) **EP 0 590 082 B1**
(45) Date of publication and mention of the grant of the patent: **27.10.1999**
(21) Application number: 92914930.0
(22) Date of filing: 11.06.1992
(51) Int. Cl.: C12N 15/11, C12N 15/12, A61K 48/00

(54) **ANTISENSE OLIGONUCLEOTIDE INHIBITION OF THE RAS GENE**
ANTISENSE-OLIGONUKLEOTID-INHIBITION DES RAS-GEN
INHIBITION PAR OLIGONUCLEOTIDE ANTI-SENS DE L'ONCOGENE RAS

(30) Priority: 14.06.1991 US 715196
(43) Date of publication of application: 06.04.1994
(73) Proprietor: ISIS PHARMACEUTICALS, INC., Carlsbad, CA 92008 (US)
(72) Inventor: MONIA, Brett, P., Carlsbad, CA 92008 (US); FREIER, Susan, M., San Diego, California 92122 (US); ECKER, David, J., Leucadia, CA 92024 (US)
(74) Representative: Hallybone, Huw George
(86) International application number: PCT/US92/05008
(87) International publication number: WO 92/22651

(56) References cited:
- US-A- 4 871 838
- US-A- 5 087 617
- ONCOGENE RESEARCH, vol.4, 1989 pages 243 - 252 BROWN, D. ET AL. 'Modulation of ras expression by anti-sense, nonionic deoxyoligonucleotide analogs'
- EMBO J 10 (5).1111-1118, May 1991 SAISON-BEHMOARAS, T. ET AL. 'SHORT MODIFIED ANTISENSE OLIGONUCLEOTIDES DIRECTED AGAINST HA - RAS POINT MUTATION INDUCE SELECTIVE CLEAVAGE OF THE MESSENGER RNA AND INHIBIT T24 CELLS PROLIFERATION.'
- JOURNAL OF BIOCHEMICAL AND BIOPHYSICAL METHODS, vol.13, 1986 pages 97 - 102 WICKSTROM, E. 'Oligodeoxynucleotide stability in subcellular extracts and culture media'
- SCI CHINA SER B CHEM LIFE SCI EARTH SCI 34 (1). 35-41, January 1991 XU, Y. ET AL. 'THE ANTISENSE C-HA-RAS OLIGODEOXYNUCLEOTIDES INHIBIT THE PROLIFERATION OF THE GASTROCARCINOMA DNA-TRANSFORMED RAT 3-3 CELLS AND REDUCE THE P21 EXPRESSION OF THE CELLS.'
- JOURNAL OF CANCER RESEARCH AND CLINICAL ONCOLOGY, vol.116, 1990 page 162 DEBUS, N. ET AL. 'Effects of antisense oligodeoxynucleotides complementary mRNA of the human c-harvey-ras oncogene on cell proliferation'
- J BIOL CHEM 267 (28).19954-19962, 5 October 1992 MONIA, B. ET AL. 'SELECTIVE INHIBITION OF MUTANT HA - RAS MRNA EXPRESSION BY ANTISENSE OLIGONUCLEOTIDES.'
- Proc. Natl. Acad. Sci. USA, Volume 85, issued February 1988, E.L. WICKSTROM et al., "Human Promyelocytic Leukemia HL-60 Cell Proliferation and c-myc Protein Expression are Inhibited by an Antisense Pentadecadeoxynucleotide Targeted Against c-myc mRNA", pages 1028-1032, see the entire document.
- Molecular and Cellular Biology, Volume 8, No. 2, issued February 1988, J.T. HOLT et al., "An Oligomer Complementary to c-myc mRNA Inhibits Proliferation of HL-60 Promyelocytic Cells and Induces Differentiation", pages 963-973, see the entire document.
- Proc. Natl. Acad. Sci. USA, Volume 86, issued May 1989, G. ANFOSSI et al., "An Oligomer Complementary to c-myb-encoded mRNA inhibits Proliferation of Human Myeloid Leukemia Cell Lines", pages 3379-3383, see the entire document.
- Anti-Cancer Drug Design, Volume 4, issued 1989, E.H. CHANG et al., "Comparative Inhibition of ras p21 Protein Synthesis with Phosphorus-modified Antisense Oligonucleotides", pages 221-232, see the entire document.
- Anti-Cancer Drug Design, Volume 3, issued 1988, D.M. TIDD et al., "Evaluation of N-ras Oncogene Anti-sense, Sense and Nonsense Sequence Methylphosphonate Oligonucleotide Analogues", pages 117-127, see the entire document.
- Science, Volume 252, issued 03 May 1991, M. KEATING et al., "Linkage of a Cardiac Arrhythmia, the Long QT Syndrome, and the Harvey ras-1 Gene", pages 704-706, see the entire document.
- Mutation Research, Volume 195, issued 1988, J. BOS, "The ras gene family and human carcinogenesis", pages 255-271, see the entire document.

## Description

### FIELD OF THE INVENTION

This invention relates to compositions and methods for the inhibition of expression of the ras gene, a naturally occurring gene which occasionally converts to an activated form which has been implicated in tumor formation. This invention is also directed to the specific inhibition of expression of the activated form of the ras gene.- This invention is further directed to the detection of both normal and activated forms of the ras gene in cells and tissues, and can form the basis for research reagents and kits both for research and diagnosis. Furthermore, this invention is directed to treatment of such conditions as arise from activation of the ras gene.

### BACKGROUND OF THE INVENTION

Alterations in the cellular genes which directly or indirectly control cell growth and differentiation are considered to be the main cause of cancer. There are some thirty families of genes, called oncogenes, which are implicated in human tumor formation. Members of one such family, the ras gene family, are frequently found to be mutated in human tumors. In their normal state, proteins produced by the ras genes are thought to be involved in normal cell growth and maturation. Mutation of the ras gene, causing an amino acid alteration at one of three critical positions in the protein product, results in conversion to a form which is implicated in tumor formation. A gene having such a mutation is said to be "activated." It is thought that such a point mutation leading to ras activation can be induced by carcinogens or other environmental factors. Over 90% of pancreatic adenocarcinomas, about 50% of adenomas and adenocarcinomas of the colon, about 50% of adenocarcinomas of the lung and carcinomas of the thyroid, and a large fraction of malignancies of the blood such as acute myeloid leukemia and myelodysplastic syndrome have been found to contain activated ras oncogenes. Overall, some 10 to 20% of human tumors have a mutation in one of the three ras genes (H-ras, K-ras, or N-ras).

It is presently believed that inhibiting expression of activated oncogenes in a particular tumor cell might force the cell back into a more normal growth habit. For example, Feramisco et al., *Nature,* 314:639-642, 1985, demonstrated that if cells transformed to a malignant state with an activated ras gene are microinjected with antibody which binds to the protein product of the ras gene, the cells slow their rate of proliferation and adopt a more normal appearance. This has been interpreted as support for the involvement of the product of the activated ras gene in the uncontrolled growth typical of cancer cells.

Antisense oligonucleotide inhibition of oncogenes has proven to be a useful tool in understanding the roles of various oncogene families. "Antisense oligonucleotides" refers to small oligonucleotides which are complementary to the "sense" or coding strand of a given gene, and as a result are also complementary to, and thus able to specifically hybridize with, the mRNA transcript of the gene. Holt et al., *Mol. Cell Biol.,* 8, 963-973, 1988, have shown that antisense oligonucleotides hybridizing specifically with mRNA transcripts of the oncogene c-myc, when added to cultured HL60 leukemic cells, inhibit proliferation and induce differentiation. Anfossi et al., *Proc. Natl. Acad. Sci.,* 86, 3379-3383, 1989, have shown that antisense oligonucleotides specifically hybridizing with mRNA transcripts of the c-myb oncogene inhibit proliferation of human myeloid leukemia cell lines. Wickstrom et al., *Proc. Nat. Acad. Sci.,* 85, 1028-1032, 1988, have shown that expression of the protein product of the c-myc oncogene as well as proliferation of HL60 cultured leukemic cells are inhibited by antisense oligonucleotides hybridizing specifically with c-myc mRNA. U.S. Patent 4,871,838 (Bos et al.) discloses oligonucleotides complementary to a mutation in codon 13 of N-ras to detect said mutation.

In all these cases, instability of unmodified oligonucleotides has been a major problem, as they are subject to degradation by cellular enzymes. PCT/US88/01024 (Zon et al.) discloses phosphorothioate oligonucleotide analogs hybridizable to the translation initiation region of the amplified c-myc oncogene to inhibit HL-60 leukemia cell growth and DNA synthesis in these cells. Tidd et al., *Anti-Cancer Drug Design,* 3, 117-127, 1988, evaluated antisense oligonucleotide methylphosphonate analogs hybridizing specifically to the activated N-ras oncogene and found that while they were resistant to biochemical degradation and were nontoxic in cultured human HT29 cells, they did not inhibit N-ras gene expression and had no effect on these cells. Chang et al., *Anti-Cancer Drug Design,* 4, 221-232, 1989, showed that both methylphosphonate and phosphorothioate analogs of oligonucleotides hybridizing specifically to mRNA transcripts of the Balb-ras gene could inhibit translation of the protein product of this gene *in vitro*. Because the antisense oligonucleotides and oligonucleotide analogs used by Chang et al. hybridize specifically with the translation initiation region of the ras gene, the binding ability of these oligonucleotides to normal (wild-type) vs. mutated (activated) ras genes was not compared.

The H-ras gene has recently been implicated in a serious cardiac arrhythmia called long Q-T syndrome, a hereditary condition which often causes sudden death if treatment is not given immediately. Frequently there are no symptoms prior to the onset of the erratic heartbeat. Whether the H-ras gene is precisely responsible for long Q-T syndrome is unclear. However, there is an extremely high correlation between inheritance of this syndrome and the presence of a particular variant of the chromosome 11 region surrounding the H-ras gene. This makes the H-ras gene an excellent indicator of increased risk of sudden cardiac death due to the long Q-T syndrome.

There is a great desire to provide compositions of matter which can modulate the expression of the ras gene, and particularly to provide compositions of matter which specifically modulate the expression of the activated form of the ras gene. It is greatly desired to provide methods of diagnosis and detection of the ras gene in animals. It is also desired to provide methods of diagnosis and treatment of conditions arising from ras gene activation. In addition, improved research kits and reagents for detection and study of the ras gene are desired.

### OBJECTS OF THE INVENTION

It is an object of the invention to provide oligonucleotides and oligonucleotide analogs which are capable of specifically hybridizing with RNA or DNA deriving from the mammalian ras gene.

It is a further object to provide oligonucleotides and oligonucleotide analogs which are capable of modulating the expression of the ras gene through antisense interaction with the mRNA product of the gene.

A further object of this invention is to provide methods of treatment of conditions arising due to mutation of the gene from the wild-type to the mutant, activated form of the ras gene.

### DETAILED DESCRIPTION OF THE INVENTION

Malignant tumors develop through a series of stepwise, progressive changes that lead to the loss of growth control characteristic of cancer cells, i.e., continuous unregulated proliferation, the ability to invade surrounding tissues, and the ability to metastasize to different organ sites. Carefully controlled *in vitro* studies have helped define the factors that characterize the growth of normal and neoplastic cells and have led to the identification of specific proteins that control cell growth and differentiation. In addition, the ability to study cell transformation in carefully controlled, quantitative *in vitro* assays has led to the identification of specific genes capable of inducing the transformed cell phenotype. Such cancer-causing genes, or oncogenes, are believed to acquire transformation-inducing properties through mutations leading to changes in the regulation of expression of their protein products. In some cases such changes occur in non-coding DNA regulatory domains, such as promoters and enhancers, leading to alterations in the transcriptional activity of oncogenes, resulting in over- or under-expression of their gene products. In other cases, gene mutations occur within the coding regions of oncogenes, leading to the production of altered gene products that are inactive, overactive, or exhibit an activity that is different from the normal (wild-type) gene product.

To date, more than 30 cellular oncogene families have been identified. These genes can be categorized on the basis of both their subcellular location and the putative mechanism of action of their protein products. The ras oncogenes are members of a gene family which encode related proteins that are localized to the inner face of the plasma membrane. ras proteins have been shown to be highly conserved at the amino acid level, to bind GTP with high affinity and specificity, and to possess GTPase activity. Although the cellular function of ras gene products is unknown, their biochemical properties, along with their significant sequence homology with a class of signal-transducing proteins known as GTP binding proteins, or G proteins, suggest that ras gene products play a fundamental role in basic cellular regulatory functions relating to the transduction of extracellular signals across plasma membranes.

Three ras genes, designated H-ras, K-ras, and N-ras, have been identified in the mammalian genome. Mammalian ras genes acquire transformation-inducing properties by single point mutations within their coding sequences. Mutations in naturally occurring ras oncogenes have been localized to codons 12, 13, and 61. The most commonly detected activating ras mutation found in human tumors is in codon 12 of the H-ras gene in which a base change from GGC to GTC results in a glycine-to-valine substitution in the GTPase regulatory domain of the ras protein product. This single amino acid change is thought to abolish normal control of ras protein function, thereby converting a normally regulated cell protein to one that is continuously active. It is believed that such deregulation of normal ras protein function is responsible for the transformation from normal to malignant growth.

The present invention provides an oligonucleotide or oligonucleotide analog specifically hybridisable with DNA or RNA deriving from the human ras gene, comprising the sequence 5'-TCCGTCATCGCTCCTCAGGG-3'.

Such oligonucleotides are conveniently and desirably presented in a pharmaceutically acceptable carrier.

In accordance with other preferred embodiments, the oligonucleotides and oligonucleotide analogs are formulated such that at least some of the linking groups between nucleotide units of the oligonucleotide comprise sulfur-containing species such as phosphorothioate moieties.

The molecules of the invention may be used in methods for modulating the expression of the human ras gene in cells or tissues and for specifically modulating the expression of the activated ras gene in cells or tissues suspected of harboring a mutation leading to such activation. Additional uses of the molecules are directed to methods of detection of the ras gene in cells or tissues and specific detection of the activated ras gene in cells or tissues suspected of harboring said mutated gene. Such methods comprise contacting cells or tissues suspected of containing the human ras gene with oligonucleotides or oligonucleotide analogs in accordance with the invention in order to interfere with the effect of or detect said gene.

The molecules of the invention may also be used in methods for diagnostics and therapeutics of animals suspected of having a mutation leading to activation of the ras gene. Such methods comprise contacting the animal or cells or tissues or a bodily fluid from the animal with oligonucleotides or oligonucleotide analogs in accordance with the invention in order to modulate the expression of this gene, to treat conditions arising from activation of this gene, or to effect a diagnosis thereof.

In the context of this invention, the term "oligonucleotide" refers to a plurality of joined nucleotide units formed from naturally-occurring bases and furanosyl groups joined by native phosphodiester bonds. This term effectively refers to naturally-occurring species or synthetic species formed from naturally-occurring subunits.

"Oligonucleotide analog," as that term is used in connection with this invention, refers to moieties which function similarly to oligonucleotides but which have non-naturally occurring portions. Thus, oligonucleotide analogs may have altered sugar moieties or inter-sugar linkages. Exemplary among these are the phosphorothioate and other sulfur-containing species which are known for use in the art. They may also comprise altered base units or other modifications consistent with the spirit of this invention.

In accordance with certain preferred embodiments, at least some of the phosphodiester bonds of the oligonucleotide have been substituted with a structure which functions to enhance the ability of the compositions to penetrate into the region of cells where the RNA whose activity is to be modulated is located, and to make the compositions more resistant to degradation by cellular enzymes. It is preferred that such linkages be sulfur-containing. It is presently preferred that such substitutions comprise phosphorothioate bonds. Others such as alkyl phosphothioate bonds, N-alkyl phosphoramidates, phosphorodithioates, alkyl phosphonates, and short chain alkyl or cycloalkyl structures may also be useful. In accordance with other preferred embodiments, the phosphodiester bonds are substituted with structures which are, at once, substantially non-ionic and non-chiral, or, alternatively, with structures that are sequence-specific and substantially chiral. Persons of ordinary skill in the art will be able to select other linkages for use in the practice of the invention.

Oligonucleotide analogs may also include species which include at least some modified base forms. Thus, purines and pyrimidines other than those normally found in nature may be so employed. In accordance with one such embodiment, one or more bases comprises 2-(amino)adenine. In other such embodiments, one or more bases comprises 2-(methylamino)adenine, 2-(alkylamino)adenine, 2-(imidazolylalkyl) adenine, 2-(aminoalkylamino)adenine or other heterosubstituted alkyladenines. Similarly, modifications on the furanose portions of the nucleotide subunits may also occur as long as the essential tenets of this invention are adhered to.

Such analogs are best described as being functionally interchangeable with natural oligonucleotides (or synthesized oligonucleotides along natural lines), but which have one or more differences from natural structure. All such analogs are comprehended by this invention so long as they function effectively to hybridize with the ras gene or mRNA deriving from it to inhibit the function or expression of the ras gene.

The oligonucleotides and oligonucleotide analogs in accordance with this invention preferably comprise from about 10 to about 30 subunits. It is more preferred that such oligonucleotides and analogs comprise from about 15 to about 25 subunits. As will be appreciated, a subunit is a base and sugar combination suitably bound to adjacent subunits through phosphodiester or other bonds.

The oligonucleotides and oligonucleotide analogs of this invention are designed to be hybridizable with messenger RNA derived from the H-ras gene. Such hybridization, when accomplished, interferes with the normal roles of the messenger RNA to cause a loss of its function in the cell. The functions of messenger RNA to be interfered with include all vital functions such as translocation of the RNA to the site for protein translation, actual translation of protein from the RNA, splicing of the RNA to yield one or more mRNA species, and possibly even independent catalytic activity which may be engaged in by the RNA. The overall effect of such interference with the RNA function is to interfere with expression of the H-ras gene. The protein products of the other mammalian ras genes, N-ras and K-ras, are identical to H-ras over the first 85 amino acids. The nucleic acid sequences of the three ras genes, while not identical, are known, and persons of ordinary skill in the art will be able to use this invention as a guide in preparing oligonucleotides or oligonucleotide analogs specifically hybridizable with the N-ras and K-ras genes.

The oligonucleotides and oligonucleotide analogs of this invention can be used in diagnostics, therapeutics and as research reagents and kits. Since the oligonucleotides and oligonucleotide analogs of this invention hybridize to the ras gene, sandwich and other assays can easily be constructed to exploit this fact. Furthermore, since the oligonucleotides and olignonucleotide analogs of this invention hybridize preferentially to the mutant (activated) form of the ras oncogene, such assays can be devised for screening of cells and tissues for ras conversion from wild-type to activated form. Such assays can be utilized for differential diagnosis of morphologically similar tumors, and for detection of increased risk of cancer stemming from ras gene activation. Provision of means for detecting hybridization of oligonucleotide or analog with the ras gene can routinely be accomplished. Such provision may include enzyme conjugation, radiolabelling or any other suitable detection systems. Kits for detecting the presence or absence of ras or activated ras may also be prepared.

The following examples illustrate the present invention and are not intended to limit the same.

### EXAMPLES

### Example 1

Oligonucleotide synthesis: Unmodified oligonucleotides were synthesized on an automated DNA synthesizer (Applied Biosystems model 380B) using standard phosphoramidate chemistry with oxidation by iodine. For phosphorothioate oligonucleotides, the standard oxidation bottle was replaced by 0.2 M solution of 3H-1,2-benzodithiole-3-one 1,1-dioxide in acetonitrile for the stepwise thiation of the phosphite linkages. The thiation wait step was increased to 68 sec and was followed by the capping step. After cleavage from the CPG column and deblocking in concentrated ammonium hydroxide at 55°C (18 hr), the oligonucleotides were purified by precipitation twice out of 0.5 M NaCl solution with 2.5 volumes ethanol. Analytical gel electrophoresis was accomplished in 20% acrylamide, 8 M urea, 454 mM Tris-borate buffer, pH=7.0. Oligonucleotides and phosphorothioates were judged from polyacrylamide gel electrophoresis to be greater than 80% full-length material.

### Example 2

ras-Luciferase Reporter Gene Assembly: The ras-luciferase reporter genes described in this study were assembled using PCR technology. Oligonucleotide primers were synthesized for use as primers for PCR cloning of the 5'-regions of exon 1 of both the mutant (codon 12) and non-mutant (wild-type) human H-ras genes. H-ras gene templates were purchased from the American Type Culture Collection (ATCC numbers 41000 and 41001) in Bethesda, MD. The oligonucleotide PCR primers 5'-ACA-TTA-TGC-TAG-CTT-TTT-GAG-TAA-ACT-TGT-GGG-GCA-GGA-GAC-CCT-GT-3' (sense), and 5'-GAG-ATC-TGA-AGC-TTC-TGG-ATG-GTC-AGC-GC-3' (antisense), were used in standard PCR reactions using mutant and non-mutant H-ras genes as templates. These primers are expected to produce a DNA product of 145 base pairs corresponding to sequences -53 to +65 (relative to the translational initiation site) of normal and mutant H-ras, flanked by NheI and HindIII restriction endonuclease sites. The PCR product was gel purified, precipitated, washed and resuspended in water using standard procedures.

PCR primers for the cloning of the *P. pyralis* (firefly) luciferase gene were designed such that the PCR product would code for the full-length luciferase protein with the exception of the amino-terminal methionine residue, which would be replaced with two amino acids, an amino-terminal lysine residue followed by a leucine residue. The oligonucleotide PCR primers used for the cloning of the luciferase gene were 5'-GAG-ATC-TGA-AGC-TTG-AAG-ACG-CCA-AAA-ACA-TAA-AG-3' (sense), and 5'-ACG-CAT-CTG-GCG-CGC-CGA-TAC-CGT-CGA-CCT-CGA-3' (antisense) , were used in standard PCR reactions using a commercially available plasmid (pT3/T7-Luc) (Clontech), containing the luciferase reporter gene, as a template. These primers were expected to yield a product of approximately 1.9 kb corresponding to the luciferase gene, flanked by HindIII and BssHII restriction endonuclease sites. This fragment was gel purified, precipitated, washed and resuspended in water using standard procedures.

To complete the assembly of the ras-luciferase fusion reporter gene, the ras and luciferase PCR products were digested with the appropriate restriction endonucleases and cloned by three-part ligation into an expression vector containing the steroid-inducible mouse mammary tumor virus promotor MMTV using the restriction endonucleases NheI, HindIII and BssHII. The resulting clone results in the insertion of H-ras 5' sequences (-53 to +65) fused in frame with the firefly luciferase gene. The resulting expression vector encodes a ras-luciferase fusion product which is expressed under control of the steroid-inducible MMTV promoter.

### Example 3

Transfection of Cells with Plasmid DNA: Transfections were performed as described by Greenberg, M.E., in Current Protocols in Molecular Biology, (F.M. Ausubel, R. Brent, R.E. Kingston, D.D. Moore, J.A. Smith, J.G. Seidman and K. Strahl, eds.), John Wiley and Sons, NY, with the following modifications. HeLa cells were plated on 60 mm dishes at 5 x 10⁵ cells/dish. A total of 10 µg of DNA was added to each dish, of which 9 µg was ras-luciferase reporter plasmid and 1 µg was a vector expressing the rat glucocorticoid receptor under control of the constitutive Rous sarcoma virus (RSV) promoter. Calcium phosphate-DNA coprecipitates were removed after 16-20 hours by washing with Tris-buffered saline [50 Mm Tris-Cl (pH 7.5), 150 mM NaCl] containing 3 mM EGTA. Fresh medium supplemented with 10% fetal bovine serum was then added to the cells. At this time, cells were pre-treated with antisense oligonucleotides prior to activation of reporter gene expression by dexamethasone.

### Example 4

Oligonucleotide Treatment of Cells: Immediately following plasmid transfection, cells were washed three times with Opti-MEM (Gibco), prewarmed to 37°C. Two ml of Opti-MEM containing 10 µg/ml N-[1-(2,3-dioleyloxy)propyl]-N,N,N,-trimethylammonium chloride (DOTMA) (Bethesda Research Labs, Gaithersburg, MD) was added to each dish and oligonucleotides were added directly and incubated for 4 hours at 37°C. Opti-MEM was then removed and replaced with the appropriate cell growth medium containing oligonucleotide. At this time, reporter gene expression was activated by treatment of cells with dexamethasone to a final concentration of 0.2 µM. Cells were harvested 12-16 hours following steroid treatment.

### Example 5

Luciferase Assays: Luciferase was extracted from cells by lysis with the detergent Triton X-100, as described by Greenberg, M.E., in Current Protocols in Molecular Biology, (F.M. Ausubel, R. Brent, R.E. Kingston, D.D. Moore, J.A. Smith, J.G. Seidman and K. Strahl, eds.), John Wiley and Sons, NY. A Dynatech ML1000 luminometer was used to measure peak luminescence upon addition of luciferin (Sigma) to 625 µM. For each extract, luciferase assays were performed multiple times, using differing amounts of extract to ensure that the data were gathered in the linear range of the assay.

### Example 6

Antisense Oligonucleotide Inhibition of ras-Luciferase Gene Expression: A series of antisense phosphorothioate oligonucleotide analogs targeted to either the H-ras translation initiation codon or the codon-12 point mutation of activated H-ras were screened using the ras-luciferase reporter gene system described in the foregoing examples. Of this initial series, six oligonucleotides were identified that gave significant and reproducible inhibition of ras-luciferase activity. The base sequences and sequence reference numbers of these oligonucleotides (all are phosphorothioate analogs) are shown in Table 1.

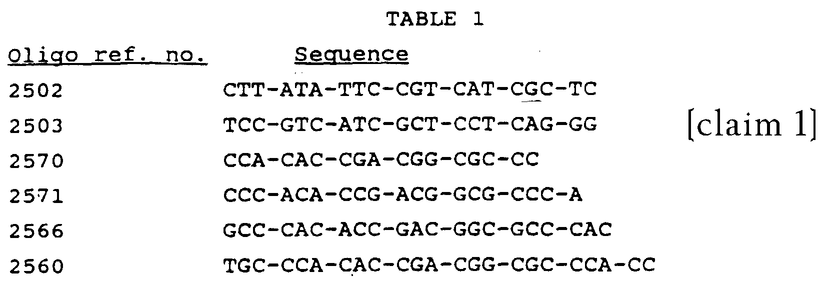

A dose-response experiment was performed in which cells expressing either the normal ras-luciferase reporter gene or the mutant ras-luciferase reporter gene were treated with increasing concentrations of the phosphorothioate oligonucleotide analog 2503. This compound is targeted to the translational initiation codon of H-ras RNA transcripts. Treatment of cells with this oligonucleotide resulted in a dose-dependent inhibition of ras-luciferase activity, displaying IC50 values of approximately 50 nM for both the normal and the mutant ras targets. The control oligonucleotide was a random phosphorothioate oligonucleotide analog, 20 bases long. Results were expressed as percentage of luciferase activity in transfected cells not treated with oligonucleotide. The observation that an oligonucleotide targeted to the ras translation initiation codon is equally effective in reducing both mutant and normal ras expression is expected since the two targets have identical sequence compositions in the region surrounding the AUG translation initiation site.

An experiment was performed in which cells expressing either the normal form or the mutant form of ras-luciferase were treated with a single dose (0.5 µM) of oligonucleotide targeted to either the translation initiation codon of H-ras or the codon-12 point mutation. The antisense phosphorothioate oligonucleotide analogs tested are shown in Table 1. The control oligonucleotide (2504) is a random phosphorothioate oligonucleotide analog, 20 bases long. Results are expressed as percentage of luciferase activity in transfected cells not treated with oligonucleotide. Compound 2503, targeted to the ras translational initiation codon, was most effective in inhibiting ras-luciferase activity. Of the three compounds targeted to the codon-12 point mutation of activated H-ras, only the 17-mer oligonucleotide 2570 displayed selectivity toward the mutated form of ras-luciferase as compared to the normal form. These results demonstrate effective antisense activity of phosphorothioate oligonucleotides targeted to ras sequences.

### EXAMPLE 7

Synthesis of 2-(amino)adenine-substituted oligonucleotides: Oligonucleotides and phosphorothioate oligonucleotide analogs will be synthesized as in Example 1, with the following exception: at positions at which a 2-(amino)adenine is desired, the standard phosphoramidite is replaced with a commercially available 2-aminodeoxyadenosine phosphoramidite (ChemGenes).

### EXAMPLE 8

2-(Amino)adenine-Modified Antisense Oligonucleotide Inhibition of ras-Luciferase Gene Expression: A series of antisense phosphorothioate oligonucleotide analogs complementary to the codon-12 point mutation of activated ras will be synthesized as described in Example 7, having a 2-(amino)adenine at the position complementary to the uracil of the mutated codon 12. Because the amino group at the 2-position of the adenine is able to hydrogen-bond with the oxygen in the 2-position on the uracil, three hydrogen bonds instead of the usual two are formed. This serves to greatly stabilize the hybridization of the 2-(amino)adenine-modified antisense oligonucleotide to the activated ras gene while destabilizing, or having no net effect, on the stability of this oligo to the wild-type codon 12, because of the modified A-G mismatch at this position.

## Claims

1. An oligonucleotide or oligonucleotide analog specifically hybridisable with DNA or RNA deriving from the human *ras* gene, comprising the sequence:

5'-TCCGTCATCGCTCCTCAGGG-3'

2. An oligonucleotide analog according to claim 1, wherein at least one of the linking groups between nucleotide units of said analog comprise sulphur-containing species.

3. An oligonucleotide analog according to claim 2, wherein the sulphur-containing species comprises a phosphorothioate.

4. A pharmaceutical composition comprising an oligonucleotide or oligonucleotide analog according to any one of claims 1 to 3 and a pharmaceutically acceptable carrier.

5. An oligonucleotide or oligonucleotide analog according to any one of claims 1 to 3 for use in medicine.

6. The use of an oligonucleotide or oligonucleotide analog according to any one of claims 1 to 3 in the preparation of a pharmaceutical composition for the modulation of the expression of the human H-*ras* gene.

7. The use of an oligonucleotide or oligonucleotide analog according to any one of claims 1 to 3 in the preparation of a composition for use in the detection of the presence of the H-*ras* gene in cells or tissues.

## Patentansprüche

1. Ein Oligonucleotid oder Oligonucleotid-Analog, das spezifisch mit einer aus dem humanen *ras* Gen stammenden DNA oder RNA hybridisierbar ist und die Sequenz 5'-TCCGTCATCGCTCCTCAGGG-3' umfaßt.

2. Ein Oligonucleotid-Analog nach Anspruch 1, in welchem mindestens eine der Verbindungsgruppen zwischen den Nucelotideinheiten des genannten Analogs eine schwefelhaltige Spezies enthält.

3. Ein Oligonucleotid-Analog nach Anspruch 2, in welchem die schwefelhaltige Spezies ein Phosphorthioat umfaßt.

4. Eine pharmazeutische Zusammensetzung, die ein Oligonucleotid oder Oligonucleotid-Analog nach einem der Ansprüche 1 bis 3 und einen pharmazeutisch verwendbaren Träger enthält.

5. Ein Oligonucleotid oder Oligonucleotid-Analog nach einem der Ansprüche 1 bis 3 zur Verwendung in der Medizin.

6. Die Verwendung eines Oligonucleotids oder Oligonucleotid-Analogs nach einem der Ansprüche 1 bis 3 bei der Herstellung einer pharmazeutischen Zusammensetzung zur Modulierung der Expression des humanen H*-ras* Gens.

7. Die Verwendung eines Oligonucleotids oder Oligonucleotid-Analogs nach einem der Ansprüche 1 bis 3 bei der Herstellung einer Zusammensetzung zur Verwendung bei der Feststellung der Anwesenheit des H*-ras* Gens in Zellen oder Geweben.

## Revendications

1. Oligonucléotide ou analogue d'oligonucléotide susceptible de s'hybrider spécifiquement avec un ADN ou un ARN issu du gêne *ras* humain, comprenant la séquence:

5'-TCCGTCATCGCTCCTCAGGG-3'

2. Analogue d'oligonucléotide selon la revendication 1, dans lequel au moins l'un des groupes de liaison entre les unités nucléotidiques dudit analogue comprend une espèce soufrée.

3. Analogue d'oligonucléotide selon la revendication 2, dans lequel l'espèce soufrée comprend un phosphorothioate.

4. Composition pharmaceutique comprenant un oligonucléotide ou un analogue d'oligonucléotide selon l'une quelconque des revendications 1 à 3 et un véhicule acceptable sur le plan pharmaceutique.

5. Oligonucléotide ou analogue d'oligonucléotide selon l'une quelconque des revendications 1 à 3, destiné à être utilisé en médecine.

6. Utilisation d'un oligonucléotide ou d'un analogue d'oligonucléotide selon l'une quelconque des revendications 1 à 3 dans la préparation d'une composition pharmaceutique pour la modulation de l'expression du gène H-*ras* humain.

7. Utilisation d'un oligonucléotide ou d'un analogue d'oligonucléotide selon l'une quelconque des revendications 1 à 3 dans la préparation d'une composition destinée à être utilisée dans la détection de la présence du gène H-*ras* dans des cellules ou des tissus.
